# EUROPEAN PATENT SPECIFICATION

(11) **EP 1 787 662 B1**
(45) Date of publication and mention of the grant of the patent: **18.03.2009**
(21) Application number: 06124039.6
(22) Date of filing: 14.11.2006
(51) Int. Cl.: A61L 2/025, A61L 2/04, A47L 15/24, B08B 3/12, B08B 3/04

(54) **Modular washing and sterilisation plant**
Modulare Wasch- und Sterilisiereinrichtung
Usine modulaire de lavage et de stérilisation

(30) Priority: 16.11.2005 IT UD20050192
(43) Date of publication of application: 23.05.2007
(73) Proprietor: International Steel Co. SpA, 31039 Riese Pio X (TV) (IT)
(72) Inventor: Zardini, Fabio, 31033 Castelfranco Veneto (TV) (IT)
(74) Representative: Petraz, Gilberto Luigi

(56) References cited:
- WO-A-01/34015
- WO-A-20/05044473
- WO-A-20/06078197
- WO-A-20/07000639
- US-A- 4 886 082

## Description

### FIELD OF THE INVENTION

The present invention concerns a washing plant, of the modular type, comprising a plurality of washer machines aligned according to a determinate disposition, connected with each other operationally, and able to perform the steps of pre-washing, washing, heat-disinfection and drying of objects, for example but not limited to medical instruments for hospital wards, operating rooms, laboratories and the pharmaceutical industry in general.

### BACKGROUND OF THE INVENTION

In the art of cleaning and sterilising objects, multiple kinds of systems are known. The following ones are of particular relevance:

WO2005/044473 and WO01/34015 disclose industrial dish washing plants comprising multiple pre-treatment steps and a washing/sterilisation step. A trolley is used for conveying the objects to be treated.

US 4 886 082 discloses a cleaning device including an ultrasonic cleaning tank.

WO 2007/000639, which constitutes state of the art according to Art. 54(3) EPC, discloses a cleaning and sterilization plant for surgical instruments comprising rinsing, chemical and heat sterilization means, whereby the objects are conveyed on a trolley. Further, the objects may be treated by ultrasound.

Usually, hospital structures comprise a plant for treating objects such as for example instruments used in the operating rooms and therefore potentially infected and not sterile, before they can be used again. This treatment usually consists of a pre-washing step, of a washing cycle proper, heat-disinfection and sterilization.

In particular, known plants are normally divided into several sectors, isolated from each other for reasons of hygiene and known respectively as the "dirty" sector, or reception, the "clean" sector, and the "sterile" sector.

In the first sector, or "dirty" sector, the dirty objects that are to be subjected to the various treatments arrive. The treatments carried out are generally: pre-washing with cold water only, possible washing in an ultrasound bath, washing in hot water and possibly detergents, the necessary rinses, heat-disinfection and final drying. In particular, heat-disinfection, which is a particular type of washing, is done with hot water, usually at a temperature comprised between about 90°C and about 93°C.

The above steps are done by means of a plurality of machines suitable to perform the above treatments.

After they have been heat-disinfected and dried, the objects pass to the second "clean" sector where they are possibly packaged and from here they are fed to sterilization machines, generally consisting of autoclaves, which sterilize them.

The objects, thus sterilized, pass to the following sterile sector where they are stored or sent again for use in the operating room.

In the field of washing plants, it is known that the pre-treatment operations are performed in a much shorter time compared with the time employed to wash, heat-disinfect and dry the objects washed.

Therefore, in known washing plants, the washing and heat-disinfection operations and the subsequent drying represent a "bottleneck" along the washing line.

Purpose of the present invention is to achieve a modular-type washing plant which, in general, has a high overall productivity and in particular which reduces the waiting times between the pre-treatment and the washing, so as to increase the productivity of the washer machines in a manner temporally coordinated with the productivity of the pre-treatment machines.

The Applicant has devised, tested and embodied the present invention to overcome the shortcomings of the state of the art and to obtain these and other purposes and advantages.

### SUMMARY OF THE INVENTION

According to the present invention, a modular washing plant, comprising a plurality of washer machines, able to perform at least a washing cycle for objects, which comprises at least a pre-treatment step and a washing and heat-disinfection step, in which there is:
at least a pre-treatment station, comprising at least two pre-treatment machines able to perform said pre-treatment step on said objects;
at least a washing station, comprising at least two washer machines disposed in parallel with respect to each other and able to perform at least said washing and heat-disinfection step on said objects;
at least a trolley able to selectively move said objects between said washer machines , to feed them alternately, one at a time;
characterized in that said pre-treatment machines comprise a pre-wash machine disposed in series with an ultrasound wash machine.

According to the present invention, the washing plant also comprises at least a trolley able to selectively move the objects from the pre-treatment machine to the washer machines, in order to feed them alternately, one at a time.

One advantage of the present invention is that it reduces the waiting times between the pre-treatment and washing, to increase the productivity of the washer machines in a manner that is temporally coordinated with the productivity of the pre-treatment machines.

Advantageously, the trolley is able to selectively translate the objects from the pre-treatment station to a selected one of the washer machines, so that the washer machines can be fed alternately by the translation system. In particular, the objects to be washed are fed by the trolley to the selected washer machine which first becomes available from the moment when the operations to pre-treat the objects have finished.

To this purpose, the washer machines are advantageously disposed aligned, or in a row, along an axis of alignment, parallel to each other, so that the trolley selectively displaces the objects to be washed along the row of washer machines in order to feed them to the washer machine that is accessible.

Advantageously, given that the time the objects remain in the pre-treatment station is very short, compared with the duration of the subsequent washing operations, they are fed, as soon as the pre-treatment is concluded, to the first available washer machine, without remaining at the exit from the corresponding pre-treatment machine; the next objects exiting from the pre-treatment station are advantageously fed, by means of the translation system, to another washer machine accessible along the row, thus increasing the productivity of the washing station.

According to another advantageous feature of the present invention, it is no longer indispensable to prepare large intermediate stores to accommodate the objects exiting from the pre-treatment station, while waiting for the washer machines to become free. In fact, since the washing station comprises a larger number of washer machines, fed alternately, than the number of pre-treatment machines, it can operate continuously on the objects exiting from the pre-treatment station.

In any case, intermediate stores for the objects exiting from the pre-treatment station can be provided, if the whole washing station is unusable, or has a reduced productivity following malfunctions or maintenance, total or partial.

Advantageously, by suitably synchronizing the operating times of the pre-treatment station and the washing station, it is possible to make the pre-treatment machines and the washer machines operate substantially continuously, and therefore without the latter representing a "bottleneck" in the washing plant.

According to another advantageous feature of the present invention, the washing plant obtained is of the modular type, very functional and simplified, since the pre-treatment and washing stations are distinct and independent. In this way, in the event of breakdowns and/or malfunctions, for example to one of the washer machines, the production of the washing cycle is not stopped, and washed objects do not accumulate at the exit from the pre-treatment station because in any case other washer machines are available.

According to another form of embodiment of the present invention, downstream of the washing station a drying station is disposed, comprising drying machines, each one connected directly to the exit of at least one relative washer machine.

Advantageously, a washing station is achieved consisting of a plurality of washer machines in parallel, disposed in series with a drying station consisting of a plurality of drying machines, also advantageously, but not only, disposed in parallel. The physical separation of the drying operation in another and distinct drying machine, distinct from the washer machine, allows to empty the latter more quickly, so as to render it available for a subsequent washing cycle and also allows to accelerate the drying operation itself. In fact, the drying chamber of each drying machine that receives the object, washed and to be dried, is itself substantially dry from the beginning of the drying operation, and therefore less energy and/or less time will be necessary to dry the object alone, which is washed and wet.

Therefore, the overall productivity of the washing plant is greatly increased.

Advantageously, therefore, according to the present invention, it is possible to achieve a modular washing plant in which the different operations of the washing cycle, such as for example pre-wash, ultrasound treatment, washing, heat disinfection and drying, are performed in independent machines, modular and selectively associated, by means of suitable means to move the objects, according to the temporal duration and the physical characteristics of said operations.

### BRIEF DESCRIPTION OF THE DRAWINGS

These and other characteristics of the present invention will become apparent from the following description of a preferential form of embodiment, given as a non-restrictive example with reference to the attached drawing wherein:
- fig. 1 is a plane schematic view of a complete pre-washing, washing, heat-disinfection and sterilization plant, comprising a washing plant according to the present invention; and
- fig. 2 is a perspective view of a washing plant according to one embodiment of the present invention.

### DETAILED DESCRIPTION OF A PREFERENTIAL FORM OF EMBODIMENT

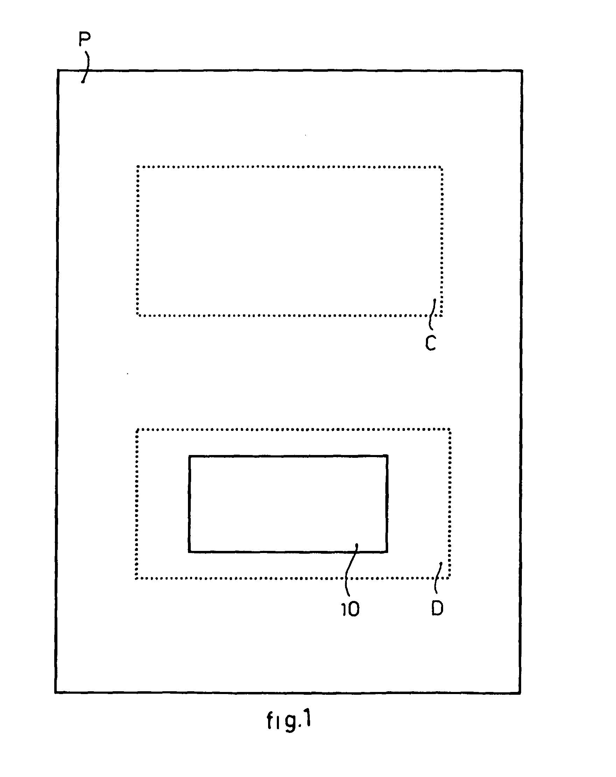

With reference to fig. 1, a washing plant 10 can be used for example in a plant P in which the complete treatment of pre-washing, washing, heat-disinfection of dirty objects is carried out, in a first zone, called "dirty" zone D, and the sterilization of the washed objects is carried out in a second zone, called "clean" zone C.

The washing plant 10 according to the present invention can be used, in particular, to perform a plurality of cycles of unitary operations to wash objects, by means of suitable machines, for pre-treating, washing, heat-disinfecting and drying objects such as, for example but not only, dirty instruments arriving from hospital wards, operating rooms, laboratories and from the pharmaceutical industry and usually contained in baskets.

By washing operations we mean both the pre-treatment operations, pre-washing and/or washing in cold water, ultrasound washing, and also the subsequent operations of washing in hot water, with chemical detergents, and also heat-disinfection, with the relative rinses and drying.

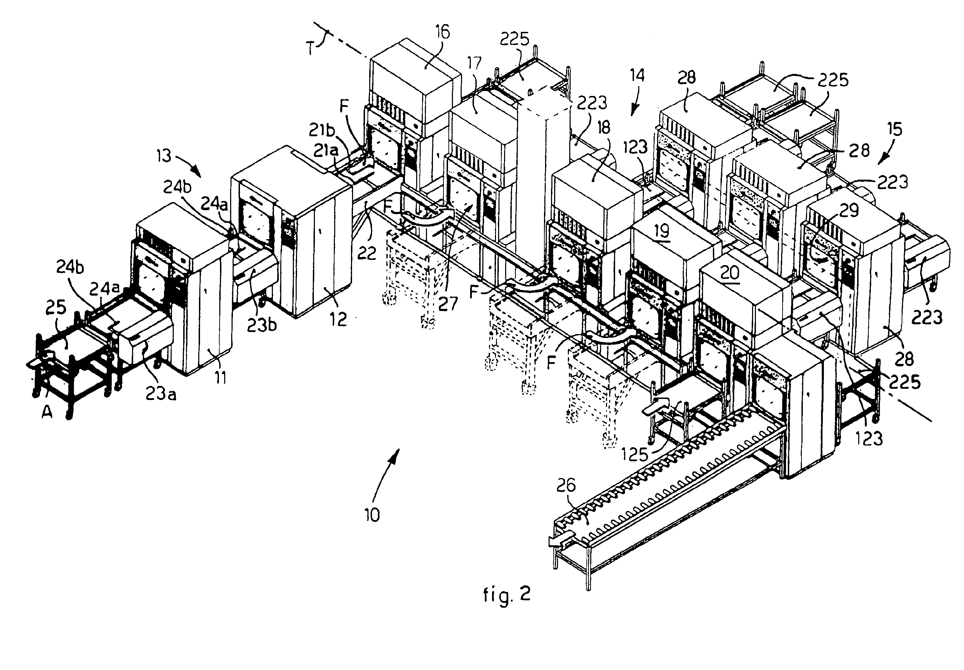

The washing plant 10 comprises at least a pre-treatment station 13 and a washing station 14 for the objects, served continuously by the pre-treatment station 13 (fig. 2). The objects are transported along the pre-treatment station 13 and the washing station 14 by means of suitable movement means, as will be shown in detail in the following part of the description.

In the pre-treatment station 13 a pre-treatment cycle is performed, which comprises one or more steps or unitary operations of pre-treatment, preliminary and preparatory to the subsequent washing and heat-disinfection, such as a pre-wash, a wash with cold water and/or an ultrasound bath.

In this case, the pre-treatment station 13 comprises at least a pre-wash machine 11 and at least an ultrasound wash machine 12, disposed in series with each other.

On the contrary, in the washing station 14, operations of washing in hot water, possibly with detergents, heat disinfection, corresponding intermediate and final rinses and the relative drying are performed.

The washing station 14 comprises a plurality of washer machines 16, 17, 18, 19, 20 able to perform the steps or operations of washing, heat-disinfection and drying of the objects, and each is provided with a suitable washing chamber 27 inside which said steps are performed.

The washer machines 16, 17, 18, 19, 20 are positioned aligned and parallel to an axis of alignment T, so that the entrance to each of them is parallel to the entrance of the other adjacent washer machines.

The objects are fed, as indicated by the arrow A, into the pre-wash machine 11 of the pre-treatment station 13 by means of said movement means which comprises a first manual trolley 25 and a subsequent trolley 23a provided with a mobile arm 24a and a conveyor belt 24b. A trolley 23b, identical to the trolley 23a, allows passage from the pre-wash machine 11 to the ultrasound wash machine 12.

According to the present invention, the movement means which moves the objects to be washed also comprises a trolley 22, 125, able to move the objects between the different washer machines 16, 17, 18, 19, 20 parallel to the axis of alignment T, so as to feed the objects to be washed to the washing chamber 27 of a selected one of the washer machines 16, 17, 18, 19, 20, for example according to the washing cycle to which the objects are to be subjected, the current availability and accessibility of the washer machines and similar parameters.

The respective washing chambers 27 are simultaneously and selectively all potentially accessible for the objects moved by the trolley 22, 125 and, of these, at least one accessible for washing is selected.

The trolley can be of the automated type, trolley 22, or of the type moved manually, trolley 125, as required.

In particular, the trolley 22, of the automated type, is able to translate automatically parallel to the axis of alignment T and is provided with a mobile arm 21a and a conveyor belt 21b, identical to the arm 24a and the conveyor belt 24b, to move the objects to be washed inside a selected washer machine 16, 17, 18, 19, 20, in a direction indicated by the arrows F, perpendicular to the axis of alignment T.

The trolley 22 may be made and moved as described in the applications for an Italian patent UD2005A000194, filed 16.11.2005, UD2005A000216, filed 21.12.2005 and UD2006A000228, filed 25.10.2006, all in the name of the present Applicant.

For example, the trolley 22 can be mounted cantilevered with respect to a supporting frame associated with the washer machines 16, 17, 18, 19, 20, or it can be made partly or completely resting on the floor in front of the washer machines 16, 17, 18, 19, 20, as shown in fig. 2.

According to another form of embodiment of the present invention, the movement of the objects to be washed is, instead, achieved by means of a fixed roller system, or a conveyor belt, a slider and similar equivalent embodiments, not shown in the drawings.

The interval of time needed to perform a pre-treatment cycle at the pre-treatment station 13 in this case is much less than that needed to perform a cycle of washing and heat-disinfection in one of the washer machines 16, 17, 18, 19, 20.

For example, the overall time for pre-washing and ultrasound washing is a few minutes, while the time required for washing, heat-definition and drying is at least about ten minutes.

In the case shown in fig. 2, the time required for three pre-treatment cycles, each consisting of a pre-wash in the machine 11 and an ultrasound wash in the machine 12, is substantially comparable, as duration, with the washing time of a single washer machine 16, 17, 18, 19, 20.

Therefore, by means of at least three washer machines 16, 17, 18, 19, 20 in parallel, where each performs the washing, heat-disinfection and drying substantially continuously, with a suitably staggered timing, the washing station 14 operates substantially continuously, receiving advantageously continuously the objects exiting from the pre-treatment station 13 and hence having a productivity comparable to the pre-treatment station 13.

The objects can be dried in the same washer machines 16, 17, 18, 19, 20 or, according to an advantageous embodiment of the invention, in drying machines downstream of the washer machines 16, 17, 18, 19, 20. In this last case, the washing plant 10 comprises a drying station 15, distinct from and disposed downstream of the washing station 14, and provided with drying machines 28.

Fig. 2 shows two washer machines 16, 17 which, apart from washing and heat-disinfection, also carry out drying operations, and three washer machines 18, 19, 20 associated with relative drying machines 28, in which the drying of the washed objects is not carried out in the washer machines 18, 19, 20 but in the relative drying machines 28. The latter receive the washed objects from the washer machines 18, 19, 20 to be dried in the relative drying chambers 29 of the drying machines 28. The passage of the objects from the washer machines 18, 19, 20 to the drying machines 28 is achieved by means of three corresponding trolleys 123, identical to the trolleys 23a, 23b, and which are positioned directly at exit from each washer machine 18, 19, 20.

Once the objects have been dried in the washing station 14, as shown in fig. 2 for the washer machines 16 and 17, or in the drying station 15, as shown in fig. 2 for the washer machines 18, 19, 20, the objects are removed by means of a trolley 223, identical to the trolleys 23a, 23b and 123, and sent to the subsequent treatment or stored, for example by means of at least a manual trolley 225, and/or a discharge roller 26, to transport them to a store.

It is clear that, as an alternative to the trolley 223, in order to discharge the dried objects it is possible to use an automated trolley which translates parallel to the axis of alignment T and is made as described in the already cited applications for an Italian patent UD2005A000194, UD2005A000216 and UD2006A000228, in the name of the present Applicant.

According to another variant, the dried objects can be discharged by means of a manual trolley, identical to the trolleys 25, 125, 225, or by means of a mechanical roller identical to the roller 26, or a conveyor belt. Furthermore, it is clear that, according to necessity, the dried objects can be discharged by hand by a suitable worker.

Therefore, by using the drying station 15, provided with drying machines 28 in parallel and disposed in correspondence with the exit of the respective washer machines 18, 19, 20, the productivity of the washing station 14 is effectively increased, and also that of the washing plant 10 as a whole. One form of embodiment of the invention which gives even greater productivity provides that all the washer machines 16, 17, 18, 19, 20 are associated with relative drying machines 28.

It is clear that modifications and/or additions of parts may be made to the washing plant 10 as described heretofore, without departing from the field and scope of the present invention.

For example, variants are provided on the type and/or number of pre-treatment machines 11, 12 in the pre-treatment station 13, to adapt to possible needs.

According to a first variant, the pre-treatment station 13 can comprise only one cold-water pre-wash machine, or only two pre-wash machines 11 disposed in series, if the pre-wash alone is sufficient.

According to a second variant, the pre-treatment station 13 comprises three or more pre-treatment machines 11, 12 disposed in series. For example, the pre-treatment station 13 can comprise two pre-wash machines 11 and an ultrasound wash machine 12 in series with each other, or a pre-wash machine 11, an ultrasound wash machine 12 and a cold-water wash machine in series with each other.

According to a third variant of the invention, the pre-treatment station 13 can comprise two or more batteries consisting of a plurality of pre-treatment machines 11, 12 disposed in series. The batteries are in turn disposed in parallel with each other. This variant is particularly advantageous when the treatment station 14 comprises a high number of washer machines 16, 17, 18, 19, 20, for example four or more.

Another variant of the present invention provides to achieve a roller or conveyor belt between the pre-treatment station 13 and the treatment station 14, which functions as a store for the objects already pre-treated and waiting to be subjected to washing and heat-disinfection. This is advantageous especially when the pre-treatment station 13 has a productivity greater than the treatment station 14, for example because one of the washer machines 16, 17, 18, 19, 20 is not usable.

## Claims

1. Modular washing plant, comprising a plurality of washer machines, able to perform at least a washing cycle for objects, which comprises at least a pre-treatment step and a washing and heat-disinfection step, in which there is:
- at least a pre-treatment station (13), comprising at least two pre-treatment machines (11, 12) able to perform said pre-treatment step on said objects;
- at least a washing station (14), comprising at least two washer machines (16, 17, 18, 19, 20) disposed in parallel with respect to each other and able to perform at least said washing and heat-disinfection step on said objects;
- at least a trolley (22, 125) able to selectively move said objects between said washer machines (16, 17, 18, 19, 20), to feed them alternately, one at a time;
**characterized in that** said pre-treatment machines comprise a pre-wash machine (11) disposed in series with an ultrasound wash machine (12).

2. Washing plant as in claim 1, **characterized in that** the trolley (22, 125) moves said objects from said pre-treatment machine (11, 12).

3. Washing plant as in claim 1 or 2, **characterized in that** the pre-wash machine (11) is disposed before the ultrasound wash machine (12).

4. Washing plant as in claim 1 or 2, **characterized in that** said pre-treatment machines comprise two pre-wash machines (11) disposed in series with each other.

5. Washing plant as in claim 1 or 2, **characterized in that** said pre-treatment station (13) comprises three pre-treatment machines (11, 12).

6. Washing plant as in claim 5, **characterized in that** said three pre-treatment machines are disposed in series with each other, of which two are pre-wash machines (11) and one is an ultrasound wash machine (12).

7. Washing plant as in claim 5, **characterized in that** said three pre-treatment machines comprise a pre-wash machine (11) disposed in series with an ultrasound wash machine (12) and with a wash machine.

8. Washing plant as in claim 1 or 2, **characterized in that** said pre-treatment station (13) comprises a plurality of batteries of pre-treatment machines (11, 12), each consisting of a plurality of pre-treatment machines (11, 12) disposed in series, wherein the batteries of pre-treatment machines of said plurality of batteries are disposed in parallel with each other.

9. Washing plant as in any claim hereinbefore, **characterized in that** said pre-treatment machines (11, 12) are associated with movement means (23a, 23b) able to move said objects along said pre-treatment station (13).

10. Washing plant as in any claim hereinbefore, **characterized in that** said washer machines (16, 17, 18, 19, 20) are also able to perform a drying step on the objects washed.

11. Washing plant as in any claim from 1 to 10, **characterized in that** it comprises a drying station (15), disposed downstream of said washing station (14) and provided with a plurality of drying machines (28), distinct from said washer machines (16, 17, 18, 19, 20) and each connected directly to the exit of a respective washer machine (16, 17, 18, 19, 20) by means of connection means (123), so that each one effects a drying operation on said washed objects exiting from said washer machines (16, 17, 18, 19, 20).

12. Washing plant as in any claim hereinbefore, **characterized in that** said washer machines (16, 17, 18, 19, 20) are disposed aligned along an axis of alignment (T).

13. Washing plant as in claim 12, **characterized in that** said trolley (22, 125) is able to transport said objects from said pre-treatment station (13) to a selected one of said washer machines (16, 17, 18, 19, 20), parallel to said axis of alignment (T) of said washing station (14), in such a manner that said washer machines (16, 17, 18, 19, 20) operate substantially continuously.

14. Washing plant as in any claim hereinbefore, **characterized in that** it comprises discharge means (223) able to pick up and discharge the washed objects at exit from the washer machines (16, 17, 18, 19, 20).

15. Washing plant as in claims 11 and 14, **characterized in that** the discharge means (223) are also able to pick up and discharge the dried objects at exit from the drying machines (28).

## Patentansprüche

1. Modulare Waschanlage, umfassend eine Vielzahl von Waschmaschinen, die dazu fähig sind, für Gegenstände mindestens einen Waschzyklus durchzuführen, der mindestens einen Vorbehandlungsschritt und einen Wasch- und Hitze-Desinfektionsschritt umfasst, in der Folgendes vorhanden ist:
- mindestens eine Vorbehandlungsstation (13), die mindestens zwei Vorbehandlungsmaschinen (11, 12) umfasst, die dazu fähig sind, den Vorbehandlungsschritt an den Gegenständen durchzuführen;
- mindestens eine Waschstation (14), die mindestens zwei Waschmaschinen (16, 17, 18, 19, 20) umfasst, die zueinander parallel angeordnet und dazu fähig sind, mindestens den Wasch- und den Hitze-Desinfektionsschritt an den Gegenständen durchzuführen;
- mindestens ein Wagen (22, 125), der dazu fähig ist, die Gegenstände selektiv zwischen den Waschmaschinen (16, 17, 18, 19, 20) zu bewegen, um sie wechselweise einzeln zu beschicken;
**dadurch gekennzeichnet, dass** die Vorbehandlungsmaschinen eine Vorwaschmaschine (11) umfassen, die mit einer Ultraschallwaschmaschine (12) in Reihe angeordnet ist.

2. Waschanlage nach Anspruch 1, **dadurch gekennzeichnet, dass** der Wagen (22, 125) die Gegenstände von der Vorbehandlungsmaschine (11, 12) bewegt.

3. Waschanlage nach Anspruch 1 oder 2, **dadurch gekennzeichnet, dass** die Vorwaschmaschine (11) vor der Ultraschallwaschmaschine (12) angeordnet ist.

4. Waschanlage nach Anspruch 1 oder 2, **dadurch gekennzeichnet, dass** die Vorbehandlungsmaschinen zwei Vorwaschmaschinen (11) umfassen, die in Reihe zueinander angeordnet sind.

5. Waschanlage nach Anspruch 1 oder 2, **dadurch gekennzeichnet, dass** die Vorbehandlungsstation (13) drei Vorbehandlungsmaschinen (11, 12) umfasst.

6. Waschanlage nach Anspruch 5, **dadurch gekennzeichnet, dass** die drei Vorbehandlungsmaschinen zueinander in Reihe angeordnet sind, von denen zwei Vorwaschmaschinen (11) sind und eine eine Ultraschallwaschmaschine (12) ist.

7. Waschanlage nach Anspruch 5, **dadurch gekennzeichnet, dass** die drei Vorbehandlungsmaschinen eine Vorwaschmaschine (11) umfassen, die mit einer Ultraschallwaschmaschine (12) und mit einer Waschmaschine in Reihe angeordnet ist.

8. Waschanlage nach Anspruch 1 oder 2, **dadurch gekennzeichnet, dass** die Vorbehandlungsstation (13) eine Vielzahl von Batterien von Vorbehandlungsmaschinen (11, 12) umfasst, die jeweils aus einer Vielzahl von Vorbehandlungsmaschinen (11, 12) bestehen, die in Reihe angeordnet sind, wobei die Batterien von Vorbehandlungsmaschinen aus der Vielzahl von Batterien parallel zueinander angeordnet sind.

9. Waschanlage nach einem der vorhergehenden Ansprüche, **dadurch gekennzeichnet, dass** den Vorbehandlungsmaschinen (11, 12) Bewegungsmittel (23a, 23b) zugeordnet sind, die dazu fähig sind, die Gegenstände entlang der Vorbehandlungsstation (13) zu bewegen.

10. Waschanlage nach einem der vorhergehenden Ansprüche, **dadurch gekennzeichnet, dass** die Waschmaschinen (16, 17, 18, 19, 20) auch dazu fähig sind, an den gewaschenen Gegenständen einen Trocknungsschritt durchzuführen.

11. Waschanlage nach einem der Ansprüche 1 bis 10, **dadurch gekennzeichnet, dass** sie eine Trocknungsstation (15) umfasst, die der Waschstation (14) nachgeschaltet ist und mit einer Vielzahl von Trocknungsmaschinen (28) ausgerüstet ist, die von den Waschmaschinen (16, 17, 18, 19, 20) getrennt und mittels Verbindungsmitteln (123) jeweils direkt mit dem Ausgang einer entsprechenden Waschmaschine (16, 17, 18, 19, 20) verbunden sind, so dass jede an den gewaschenen Gegenständen, die aus den Waschmaschinen (16, 17, 18, 19, 20) herauskommen, einen Trocknungsvorgang durchführt.

12. Waschanlage nach einem der vorhergehenden Ansprüche, **dadurch gekennzeichnet, dass** die Waschmaschinen (16, 17, 18, 19, 20) entlang einer Ausrichtungsachse (T) ausgerichtet angeordnet sind.

13. Waschanlage nach Anspruch 12, **dadurch gekennzeichnet, dass** der Wagen (22, 125) dazu fähig ist, die Gegenstände von der Vorbehandlungsstation (13) zu einer Ausgewählten der Waschmaschinen (16, 17, 18, 19, 20) parallel zur Ausrichtungsachse (T) der Waschstation (14) in einer solchen Weise zu transportieren, dass die Waschmaschinen (16, 17, 18, 19, 20) im Wesentlichen durchgehend betrieben werden.

14. Waschanlage nach einem der vorhergehenden Ansprüche, **dadurch gekennzeichnet, dass** sie Austragsmittel (223) umfasst, die dazu fähig sind, die gewaschenen Gegenstände beim Herauskommen aus den Waschmaschinen (16, 17, 18, 19, 20) aufzunehmen und auszutragen.

15. Waschanlage nach den Ansprüchen 11 und 14, **dadurch gekennzeichnet, dass** die Austragsmittel (223) auch dazu fähig sind, die getrockneten Gegenstände beim Herauskommen aus den Trocknungsmaschinen (28) aufzunehmen und auszutragen.

## Revendications

1. Installation modulaire de lavage, comprenant une pluralité de machines de lavage, aptes à réaliser au moins un cycle de lavage pour des objets, comprenant au moins une étape de prétraitement et une étape de lavage et de désinfection thermique, comprenant :
- au moins une station de prétraitement (13), comprenant au moins deux machines de prétraitement (11, 12) aptes à réaliser ladite étape de prétraitement sur lesdits objets ;
- au moins une station de lavage (14), comprenant au moins deux machines de lavage (16, 17, 18, 19, 20) disposées de manière mutuellement parallèle et aptes à réaliser au moins ladite étape de lavage et de désinfection thermique sur lesdits objets ;
- au moins un chariot (22, 125) apte à déplacer de manière sélective lesdits objets entre lesdites machines de lavage (16, 17, 18, 19, 20), pour les alimenter de manière alternée, une à la fois ;
**caractérisée en ce que** lesdites machines de prétraitement comprennent une machine de prélavage (11) disposée en série avec une machine de lavage par ultrasons (12).

2. Installation de lavage selon la revendication 1, **caractérisée en ce que** le chariot (22, 125) déplace lesdits objets depuis ladite machine de prétraitement (11, 12).

3. Installation de lavage selon la revendication 1 ou 2, **caractérisée en ce que** la machine de prélavage (11) est disposée avant la machine de lavage par ultrasons (12).

4. Installation de lavage selon la revendication 1 ou 2, **caractérisée en ce que** lesdites machines de prétraitement comprennent deux machines de prélavage (11) disposées mutuellement en série.

5. Installation de lavage selon la revendication 1 ou 2, **caractérisée en ce que** la station de prétraitement (13) comprend trois machines de prétraitement (11, 12).

6. Installation de lavage selon la revendication 5, **caractérisée en ce que** lesdites trois machines de prétraitement sont disposées mutuellement en série, deux d'entre elles étant des machines de prélavage (11) et une étant une machine de lavage par ultrasons (12).

7. Installation de lavage selon la revendication 5, **caractérisée en ce que** lesdites trois machines de prétraitement comprennent une machine de prélavage (11) disposée en série avec une machine de lavage par ultrasons (12) et avec une machine de lavage.

8. Installation de lavage selon la revendication 1 ou 2, **caractérisée en ce que** ladite station de prétraitement (13) comprend une pluralité de batteries de machines de prétraitement (11, 12), chacune consistant en une pluralité de machines de prétraitement (11, 12) disposées en série, dans laquelle les batteries de machines de prétraitement de ladite pluralité de batteries sont disposées de manière mutuellement parallèle.

9. Installation de lavage selon l'une quelconque des revendications précédentes, **caractérisée en ce que** lesdites machines de prétraitement (11, 12) sont associées à des moyens de déplacement (23a, 23b) aptes à déplacer lesdits objets le long de ladite station de prétraitement (13).

10. Installation de lavage selon l'une quelconque des revendications précédentes, **caractérisée en ce que** lesdites machines de lavage (16, 17, 18, 19, 20) sont également aptes à réaliser une étape de séchage sur les objets lavés.

11. Installation de lavage selon l'une quelconque des revendications 1 à 10, **caractérisée en ce qu'**elle comprend une station de séchage (15), disposée en aval de ladite station de lavage (14) et munie d'une pluralité de machines de séchage (28), distinctes desdites machines de lavage (16, 17, 18, 19, 20) et chacune reliée directement à la sortie d'une machine de lavage respective (16, 17, 18, 19, 20) au moyen de moyens de liaison (123), afin que chacune effectue une opération de séchage sur lesdits objets lavés sortant desdites machines de lavage (16, 17, 18, 19, 20).

12. Installation de lavage selon l'une quelconque des revendications précédentes, **caractérisée en ce que** lesdites machines de lavage (16, 17, 18, 19, 20) sont disposées de manière alignée le long d'un axe d'alignement (T).

13. Installation de lavage selon la revendication 12, **caractérisée en ce que** ledit chariot (22, 125) est apte à transporter lesdits objets depuis ladite station de prétraitement (13) jusqu'à l'une desdites machines de lavage (16, 17, 18, 19, 20), de manière parallèle audit axe d'alignement (T) de ladite station de lavage (14), de telle manière que lesdites machines de lavage (16, 17, 18, 19, 20) fonctionnent de manière sensiblement continue.

14. Installation de lavage selon l'une quelconque des revendications précédentes, **caractérisée en ce qu'**elle comprend des moyens d'évacuation (223) aptes à recueillir et à évacuer les objets lavés à la sortie des machines de lavage (16, 17, 18, 19, 20).

15. Installation de lavage selon les revendications 11 et 14, **caractérisée en ce que** les moyens d'évacuation (223) sont également aptes à recueillir et à évacuer les objets séchés à la sortie des machines de séchage (28).
